# EUROPEAN PATENT SPECIFICATION

(11) **EP 3 685 753 B1**
(45) Date of publication and mention of the grant of the patent: **30.10.2024**
(21) Application number: 20152280.2
(22) Date of filing: 16.01.2020
(51) Int. Cl.: A61B 8/00

(54) **ULTRASOUND IMAGING APPARATUS AND METHOD OF CONTROLLING THE SAME**
ULTRASCHALLBILDGEBUNGSVORRICHTUNG UND VERFAHREN ZUR STEUERUNG DAVON
APPAREIL D'IMAGERIE À ULTRASONS ET PROCÉDÉ DE COMMANDE CORRESPONDANT

(30) Priority: 16.01.2019 KR 20190005543
(43) Date of publication of application: 29.07.2020
(73) Proprietor: Samsung Medison Co., Ltd., Hongcheon-gun, Gangwon-do, 25108 (KR)
(72) Inventor: PARK, Jun Sung, 06131 Seoul (KR); BANG, Won Chul, 13643 Gyeonggi-do (KR); YI, Jong Hyon, 16903 Gyeonggi-do (KR); PARK, Moon Ho, 18452 Gyeonggi-do (KR); JEONG, Yeonmo, 06994 Seoul (KR)
(74) Representative: D'Halleweyn, Nele Veerle Trees Gertrudis

(56) References cited:
- EP-A1- 2 564 788
- EP-A1- 2 977 012
- JP-A- 2000 300 557
- KR-A- 20100 087 521
- US-A1- 2004 019 270

## Description

### BACKGROUND

### 1. Field

The present disclosure relates to an ultrasound imaging apparatus for storing and outputting an ultrasound diagnosis process, and a method of controlling the same.

### 2. Description of the Related Art

Ultrasound imaging apparatuses operate to irradiate an ultrasound signal through the surface of an object to a target site inside the object and receive an ultrasound echo signal reflected from the target site to obtain a cross-sectional image of a soft tissue or bloodstream in a non-invasive manner. The ultrasound imaging apparatuses are smaller in size and cheaper compared to other image diagnosis devices (e.g., X-ray diagnosis device, computerized tomography (CT) scanner, magnetic resonance imaging (MRI), nuclear medicine diagnosis device, etc.). In addition, the ultrasound imaging apparatuses may enable real-time display of a diagnosis image and be highly safe because there is no risk of exposure to X-rays. Thus, these ultrasound diagnosis devices are widely used in diagnosis at obstetrics and gynecology, diagnosis for the heart and abdomen, and urology diagnosis.

The ultrasound imaging apparatus includes an ultrasound probe to transmit an ultrasound signal to an object and receive an ultrasound echo signal reflected from the object to obtain an ultrasound image of the object and a main body to generate an image of an internal state of the object using the ultrasound echo signal received from the ultrasound probe.

Meanwhile, such an ultrasound imaging apparatus diagnoses an object while moving the probe due to the nature thereof. In this regard, a technology for storing the area passed by the ultrasound probe has been proposed, but there is no technology for determining whether a specific area has been used for diagnosis, which causes inconvenience in identifying an area required for diagnosis each time the object is diagnosed. Accordingly, in order to enhance the efficiency of disease diagnosis of an object using an ultrasound imaging apparatus, research on a technology of storing an area required for a diagnosis to be matched with the object is needed.

EP2977012A1 describes an ultrasound imaging apparatus and a controlling method thereof, the ultrasound imaging apparatus includes a receiving unit receiving an imaging signal of an object, a display unit displaying a diagnostic imaging on a first section based on the imaging signal and displaying an image indicator indicating a target part of the object on a second section, and a controlling unit synchronizing the diagnostic imaging with a reference image corresponding to the diagnostic imaging.

### SUMMARY

Therefore, it is an object of the present disclosure to provide an ultrasound imaging apparatus capable of enhancing the diagnosis efficiency of an object by learning an area used for a diagnosis of an object and providing information about the area at a later time, and a method of controlling the same.

Additional aspects of the disclosure will be set forth in part in the description which follows and, in part, will be obvious from the description, or may be learned by practice of the disclosure.

Therefore, it is an aspect of the present disclosure to provide an ultrasound imaging apparatus including: an output: a probe; a sensor configured to acquire position information of the probe; a storage configured to store a model of an object that is derived in advance; and a controller configured to determine an area of the model corresponding to a position of the probe when acquiring an ultrasound image of the object, determine a diagnosis area used for diagnosis of the object among the scan areas on the basis of at least one of a quality parameter of the ultrasound image or the position information of the probe, and output the scan area and the diagnosis area to correspond to the model on the output.

The controller determines the area of the model corresponding to the position of the probe when a movement speed of the probe is less than a predetermined value as the diagnosis area.

The controller may determine the area of the model corresponding to the position of the probe when a proportion of shade included in the ultrasound image acquired by the probe is less than a predetermined value as the diagnosis area.

The controller may output each of the scan area and the diagnosis area to correspond to the object in a different form.

The controller may learn a type of the object and the diagnosis area corresponding to the model, and determine a reference diagnosis area corresponding to the object.

The controller may output the reference diagnosis area to correspond to the model of the object, and guide the position of the ultrasound probe p to match with the reference diagnosis area on the basis of a positional relationship between the object and the probe.

The ultrasound imaging apparatus may further include an input configured to receive a region of interest (ROI) of a user, wherein the storage may store at least one image of the object used for deriving the model, and the controller may output at least one of the ultrasound image, the model or the image of the object corresponding to the ROI of the user.

The controller may determine an area of the ultrasound image corresponding to the ROI set in the image of the object as an object area.

It is another aspect of the present disclosure to provide a method of controlling an ultrasound imaging apparatus, the method including: acquiring position information of a probe; storing a model of an object that is derived in advance; determining an area of the model corresponding to a position of the probe when acquiring an ultrasound image of the object; determining a diagnosis area used for diagnosis of the object among the scan areas on the basis of at least one of a quality parameter of the ultrasound image or the position information of the probe; and outputting the scan area and the diagnosis area to correspond to the model.

The determining of the diagnosis area includes determining the area of the model corresponding to the position of the probe when a movement speed of the probe is less than a predetermined value as the diagnosis area.

The determining of the diagnosis area may include determining the area of the model corresponding to the position of the probe when a proportion of shade included in the ultrasound image acquired by the probe is less than a predetermined value as the diagnosis area.

The outputting of the scan area and the diagnosis area to correspond to the model may include outputting each of the scan area and the diagnosis area to correspond to the object in a different form.

The method may further include learning a type of the object and the diagnosis area corresponding to the model, and determining a reference diagnosis area corresponding to the object.

The method may further include: outputting the reference diagnosis area to correspond to the model of the object; and guiding the position of the ultrasound probe p to match with the reference diagnosis area on the basis of a positional relationship between the object and the probe.

The method may further include: receiving a region of interest (ROI) of a user; storing at least one image of the object used for deriving the model; and outputting at least one of the ultrasound image, the model or the image of the object corresponding to the ROI of the user.

The outputting of the at least one of the ultrasound image, the model or the image of the object corresponding to the ROI of the user may include determining an area of the ultrasound image corresponding to the ROI set in the image of the object as an object area.

### BRIEF DESCRIPTION OF THE DRAWINGS

These and/or other aspects of the disclosure will become apparent and more readily appreciated from the following description of the embodiments, taken in conjunction with the accompanying drawings of which:
FIG. 1 is a diagram illustrating an external appearance of an ultrasound imaging apparatus according to an embodiment of the disclosure;
FIG. 2 is a control block diagram illustrating an ultrasound imaging apparatus according to an embodiment of the disclosure;
FIG. 3 is a control block diagram in detail illustrating a configuration of a main body of an ultrasound imaging apparatus according to an embodiment;
FIG. 4 is a control block diagram schematically illustrating a configuration of a main body of an ultrasound imaging apparatus according to an embodiment;
FIG. 5A is a diagram for describing a scan area according to an embodiment;
FIG. 5B is a diagram for describing a diagnosis area according to an embodiment;
FIG. 6 is a diagram for describing an operation of guiding a user on the basis of a reference diagnosis area according to an embodiment;
FIG. 7A is a diagram for describing an operation of determining a scan area and a diagnosis area according to an embodiment;
FIG. 7B is a diagram for describing a relationship between an ultrasound image and an image quality according to an embodiment;
FIG. 7C is a diagram for describing a relationship between an ultrasound image and a shadow according to an embodiment;
FIG. 8A is a diagram for describing an operation of outputting a scan area according to an embodiment;
FIG. 8B is a diagram for describing an operation of outputting a diagnosis area according to an embodiment;
FIG. 9A is a diagram for describing an operation associated with an ROI according to an embodiment;
FIG. 9B is a diagram for describing an operation associated with an ROI according to an embodiment; and
FIG. 10 is a flowchart according to an embodiment.

### DETAILED DESCRIPTION

Like numerals refer to like elements throughout the specification. Not all elements of embodiments of the present disclosure will be described, and description of what are commonly known in the art or what overlap each other in the embodiments will be omitted. The terms as used throughout the specification, such as "∼ part", "∼ module", "∼ member", "∼ block", etc., may be implemented in software and/or hardware, and a plurality of "∼ parts", "∼ modules", "∼ members", or "∼ blocks" may be implemented in a single element, or a single "∼ part", "∼ module", "∼ member", or "∼ block" may include a plurality of elements.

It will be further understood that the term "connect" or its derivatives refer both to direct and indirect connection, and the indirect connection includes a connection over a wireless communication network. It will be further understood that the terms "comprises" and/or "comprising," when used in this specification, specify the presence of stated features, integers, steps, operations, elements, and/or components, but do not preclude the presence or addition of one or more other features, integers, steps, operations, elements,

Further, when it is stated that one member is "on" another member, the member may be directly on the other member or a third member may be disposed therebetween.

Although the terms "first," "second," "A," "B," etc. may be used to describe various components, the terms do not limit the corresponding components, but are used only for the purpose of distinguishing one component from another component.

As used herein, the singular forms "a," "an" and "the" are intended to include the plural forms as well, unless the context clearly indicates otherwise.

Reference numerals used for method steps are just used for convenience of explanation, but not to limit an order of the steps. Thus, unless the context clearly dictates otherwise, the written order may be practiced otherwise.

Hereinafter, the principles and embodiments of the disclosure will be described with reference to the accompanying drawings.

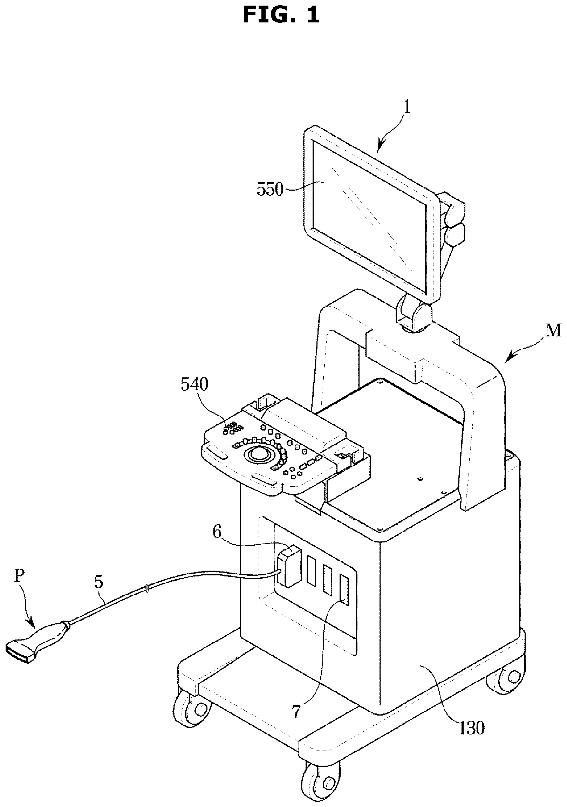

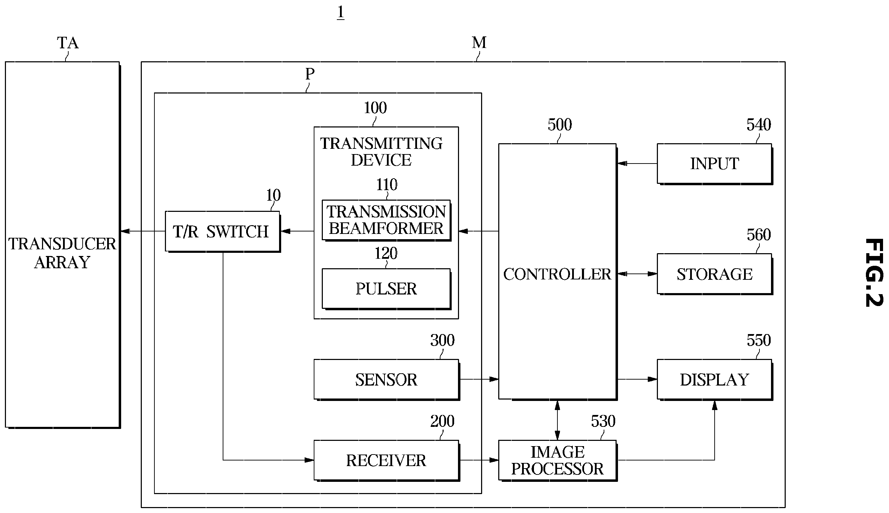

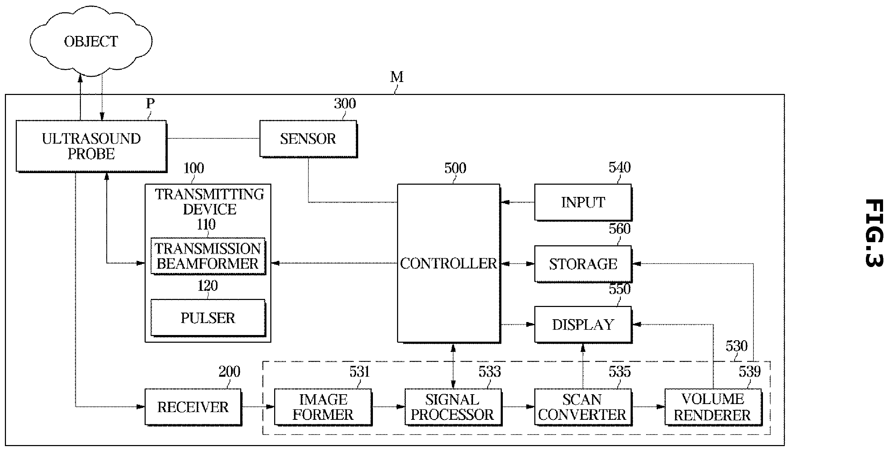

FIG. 1 is a diagram illustrating an external appearance of an ultrasound imaging apparatus 1 according to an embodiment of the disclosure. FIG. 2 is a control block diagram of the ultrasound imaging apparatus 1 according to an embodiment of the disclosure. FIG. 3 is a control block diagram in detail illustrating a configuration of a main body of the ultrasound imaging apparatus 1 according to an embodiment.

Referring to FIG. 1, the ultrasound imaging apparatus 1 includes an ultrasound probe P that transmits ultrasound to an object, receives an ultrasound echo signal from the object, and converts the ultrasound signal into an electrical signal, and a main body M connected to the ultrasound probe P and having an input 540 and a display 550 to display an ultrasound image. The ultrasound probe P is connected to the main body M of the ultrasound imaging apparatus through a cable 5 to receive various signals required for controlling the ultrasound probe P, or transmit an analog signal or digital signal corresponding to an ultrasound eco signals received by the ultrasound probe P to the main body M. However, the embodiment of the ultrasound probe P is not limited thereto, and the ultrasound probe P may be implemented as a wireless probe to transmit and receive signals through a network formed between the ultrasound probe P and the main body M.

In addition, the ultrasound probe p may include a sensor 300 that acquires position information of the ultrasound probe p.

One end of the cable 5 is connected to the ultrasound probe P, and a connector 6 is provided at the other end of the cable 5 to be coupled to or separated from a slot 7 of the main body M. The main body M and the ultrasound probe P may exchange control commands or data therebetween using the cable 5. For example, when a user inputs information about the depth of focus, the size or shape of the aperture, or the steering angle through the input 540, the information is transmitted to the ultrasound probe P through the cable 5, and is used for transmission and reception beamforming of a transmitting device 100 and a receiver 200. Alternatively, when the ultrasound probe P is implemented as a wireless probe as described above, the ultrasound probe P is connected to the main body M through a wireless network, rather than the cable 5. Even when the ultrasound probe p is connected to the main body M through a wireless network, the main body M and the ultrasound probe P may exchange the above-described control commands or data. The main body M may include a controller 500, an image processor 530, an input 540, and a display 550 as shown in FIG. 2.

The controller 500 controls the overall operation of the ultrasound imaging apparatus 1. In detail, the controller 500 generates a control signal for controlling components of the ultrasound imaging apparatus 1, for example, the transmitting device 100, a T/R switch 10, the receiver 200, the image processor 530, and the display 550 shown in FIG. 2 and controls the operations of the components. Although the ultrasound imaging apparatus according to the embodiment shown in FIG. 2 is illustrated as including transmission and reception beamformers in the ultrasound probe P, the transmission and reception beamformers may be included in the main body rather than in the ultrasound probe P.

The controller 500 calculates a delay profile of a plurality of ultrasound transducer elements 60 constituting an ultrasound transducer array TA, and on the basis of the calculated delay profile, calculates a time delay value according to the distance difference between the plurality of ultrasound transducer elements 60 included in the ultrasound transducer array TA and the focal point of an object. The controller 500 accordingly controls the transmission and reception beamformers to generate transmission and reception signals.

The transducer array TA may be configured to be included in the main body M or the ultrasound probe p.

In addition, the controller 500 may generates a control command for each component of the ultrasound imaging apparatus 1 according to a user's instruction or command input through the input 540 and control the ultrasound imaging apparatus 1.

The image processor 530 generates an ultrasound image of a target site inside the object on the basis of ultrasound signals focused through the receiver 200.

Referring to FIG. 3, the image processor 530 may include an image former 531, a signal processor 533, a scan converter 535, a storage 560, and a volume renderer 539.

The image former 531 generates a coherent 2D image or 3D image of a target site inside the object on the basis of the ultrasound signals focused through the receiver 200.

The signal processor 533 converts coherent image information formed by the image former 531 into ultrasound image information according to a diagnosis mode, such as a brightness mode (B-mode) or a Doppler mode (D-mode). For example, when the diagnosis mode is set to the B-mode, the signal processor 533 performs processing, such as A/D conversion processing, and generates ultrasound image information for a B-mode image in real time. In addition, when the diagnosis mode is set to the D-mode, the signal processor 533 extracts phase change information from ultrasound signals, calculates information of a blood flow corresponding to each point of a photography section, such as speed, power, and dispersion, and generates ultrasound image information for a D-mode image in real time.

The scan converter 535 converts the converted ultrasound image information received from the signal processor 533 or the converted ultrasound image information stored in the storage 560 into a general video signal for the display 550, and transmits the video signal to the volume renderer 539.

The storage 560 temporarily or non-temporarily stores the ultrasound image information converted by the signal processor 533.

In addition, the storage 560 may store an image of the object and a model of the object.

The volume renderer 539 performs volume rendering on the basis of the video signal transmitted from the scan converter 535, corrects the rendered image information to generate a final resultant image, and then transmits the generated final resultant image to the display 550.

The input 540 is provided to allow a user to input a command regarding the operation of the ultrasound imaging apparatus 1. The user may input or set an ultrasound diagnosis start command, a diagnosis mode selection command, such as a brightness mode (B-mode), a motion mode (M-mode), a Doppler mode (D-mode), an elastography mode (E-mode), and a three dimensional mode (3D mode), region of interest (ROI) setting information including the size and position of an ROI, and the like through the input 540.

The B-mode is provided to display a cross sectional image of an internal state of an object, and represent the magnitude of ultrasound echo waves in brightness of the ultrasound image. The B-mode ultrasound image is constructed on the basis of information obtained from tens to hundreds of scan lines.

The M-mode is provided to display a change of biometric information (e.g., luminance information) of a specific portion (M line) of a cross-sectional image of an object (a B-mode image) over time. In general, the B-mode image and the M-mode image are displayed on one screen at the same time such that a user performs accurate diagnosis by comparing and analyzing the two data.

The D-mode image is an ultrasound image using the Doppler effect that the frequency of sound emitted from a moving object changes. The mode using the Doppler effect may be further classified into a power Doppler Imaging (PDI) mode, a color flow (S Flow) mode, and a DPDI mode.

The PDI mode is provided to represent the degree of a Doppler signal or the number of structures (red blood cells in the blood) in an image. The PDI mode, as being less sensitive to an incident angle, has no aliasing and less attenuation of image. In addition, since the PDI mode records reflected Doppler energy, it has a high sensitivity enough to detect even small blood vessels and slow blood flow.

The S Flow mode provides a power image (power Doppler imaging: (PDI)) in which the power of a Doppler signal is represented in a two-dimensional distribution, and provides a velocity image in which the velocity of a Doppler signal is represented in a two-dimensional distribution. The S flow mode image may visualize a blood flow in real time while representing a wide range of blood flow conditions, from a high velocity blood flow in large vessels to a low velocity blood flow in small vessels.

The DPDI mode image refers to a direction image in which direction information of a Doppler signal in a PDI mode is represented in a two-dimensional distribution. Therefore, the information about the blood flow is more accurately detected compared to PDI. In addition, an M mode image may be generated with respect to the Doppler mode image.

The E-mode refers to a method of acquiring an ultrasound elasticity image of an object using elastography. Elastography analyzes that a harder structure, such as a malignant mass, has a lower tissue elasticity and thus exhibits a small degree of tissue denaturation according a pressure. An elastography image refers to an image in which stiffness of a tissue is quantitatively represented. In particular, elastography is widely used in the field of cervix test, breast cancer test, prostate cancer test, and the like.

The 3D mode image generally refers to an image in which a geometrical solid or space including X, Y, and Z values corresponding to depth, width, and height is represented, and may refer to a series of images representing a stereoscopic effect or a three-dimensional effect in a three-dimensional form. For example, using the three-dimensional effect of the 3D mode, the user may display the shape of a fetal face and show the fetal face to parents.

Meanwhile, the operation of the present disclosure may be implemented in an ultrasound contrast image obtained by entering an ultrasound contrast mode, but the present disclosure is not limited to the corresponding mode as long as an image of an object is derived on the basis of a change of an image signal.

The input 540 may include various devices by which a user may input data, instructions, or commands, such as a keyboard, a mouse, a trackball, a tablet PC, or a touch screen module.

The display 550 displays a menu or guide for ultrasound diagnosis and an ultrasound image obtained during an ultrasound diagnosis. The display 550 displays an ultrasound image of a target site inside an object generated by the image processor 530. The ultrasound image displayed on the display 550 may be an ultrasound image in a B-mode, an ultrasound image in an E-mode, or a 3D stereoscopic ultrasound image. The display 550 may display various ultrasound images according to the above-described modes.

The display 550 may be implemented by various known display methods such as a cathode ray tube (CRT) and a liquid crystal display (LCD).

The ultrasound probe P according to the embodiment may include the transducer array TA, the T/R switch 10, the transmitting device 100, the receiver 200, and the sensor 300 as shown in FIG. 2. The transducer array TA is provided at the end of the ultrasound probe P. The ultrasound transducer array TA refers to a plurality of ultrasound transducer elements 60 arranged in a one-dimensional or two-dimensional array. The transducer array TA may vibrate according to a pulse signal or alternating current signal applied thereto to generate ultrasound waves. The generated ultrasound waves are directed to a target site inside an object. In this case, the ultrasound waves generated by the TA may be directed by focusing on a plurality of target sites inside of the object. That is, the generated ultrasound waves may be directed to the plurality of target sites by multi-focusing.

The ultrasound waves generated by the transducer array TA are reflected from the target site inside the object to return to the transducer array TA. The transducer array TA receives ultrasound echo waves (an echo signal) reflected from the target site. When the ultrasound echo waves reach the transducer array TA, the transducer array TA vibrates with a predetermined frequency corresponding to a frequency of the ultrasound echo waves to output alternating current of a frequency corresponding to the vibration frequency of the transducer array TA. Accordingly, the transducer array TA may convert the received ultrasound echo signal into a predetermined electrical signal. Since each ultrasound element 60 receives an ultrasound echo signal and outputs an electrical signal into which the ultrasound echo signal has been converted, the transducer array TA may output electrical signals of a plurality of channels.

The ultrasound transducer may be, for example, any one of a magnetostrictive ultrasound transducer using a magnetostrictive effect of a magnetic body, a piezoelectric ultrasound transducer using a piezoelectric effect of a piezoelectric material, and a capacitive micromachined ultrasound transducer (cMUT), which transmits and receives ultrasound waves using vibration of several hundreds or several thousands of micromachined thin films. In addition, other kinds of transducers that generate ultrasound waves according to an electrical signal or generate an electrical signal according to ultrasound waves may also be used as the ultrasound transducer.

For example, the ultrasound transducer element 60 may include a piezoelectric vibrator or a thin film. When alternating current is applied to piezoelectric vibrators or thin films of the ultrasound transducers from a power source, the piezoelectric vibrators or thin films vibrate with a predetermined frequency according to applied alternating current and ultrasound waves of the predetermined frequency are generated according to the vibration frequency. On the other hand, when ultrasound echo waves of the predetermined frequency reach the piezoelectric vibrators or thin films, the piezoelectric vibrators or thin films vibrate according to the ultrasound echo waves. In this regard, the piezoelectric vibrators or thin films output alternating current of a frequency corresponding to the vibration frequency thereof.

The transmitting device 100 applies a transmission pulse to the transducer array TA such that the transducer array TA transmits an ultrasound signal to a target site inside the object. The transmitting device 100 may include a transmission beamformer and a pulser. The transmission beamformer 110 forms a transmission signal pattern according to a control signal of the controller 500 of the main body M and outputs the transmission signal pattern to the pulser 120. The transmission beamformer 110 forms a transmission signal pattern on the basis of a time delay value for the respective ultrasound transducer elements 60 constituting the ultrasound transducer array TA calculated by the controller 500, and transmits the transmission signal pattern to the pulser 120.

The receiver 200 performs a predetermined processing on ultrasound echo signals received from the transducer array TA and performs reception beamforming. The receiver 200 may include a reception signal processor and a reception beamformer.

The receiver 200, upon receiving a signal from the transducer, may perform image processing and signal processing on the signal. An electrical signal converted by the transducer array TA is input to the reception signal processor. The reception signal processor, before performing a signal processing or a time delay processing on the electrical signal into which the ultrasound echo signal has been converted, may amplify the electrical signal, adjust a gain, or compensate for attenuation according to a depth. In more detail, the reception signal processor may include a low noise amplifier (LNA) for reducing the noise of the electrical signal input from the ultrasound transducer array TA and a variable gain amplifier (VGA) for controlling the gain according to the input signal. The VGA may be implemented as a time gain compensation (TGC) for compensating a gain according to a distance from a focal point, but the implementation of the reception signal processor is not limited thereto.

The reception beamformer performs beamforming on the electrical signal input from the reception signal processor. The reception beamformer strengthens the signal intensity by superposition of the electrical signal input from the reception signal processor. The signal subjected to the beamforming in the reception beamformer is converted into a digital signal through an analog-digital converter and is transmitted to the image processor 530 of the main body M. When the analog-to-digital converter is provided in the main body M, the analog signal subjected to the beamforming in the reception beamformer may be transmitted to the main body M and converted into a digital signal in the main body M. Alternatively, the reception beamformer may be a digital beamformer. A digital beamformer may include a storage for sampling and storing analog signals, a sampling period controller 500 for controlling a sampling period, an amplifier for adjusting a sample size, an anti-aliasing low pass filter for preventing aliasing before sampling, a bandpass filter for selecting a desired frequency band, an interpolation filter for increase a sampling rate during beamforming, and a high-pass filter for removing DC components or low frequency band signals.

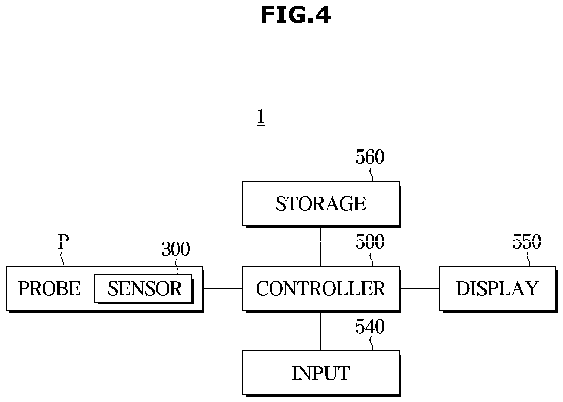

FIG. 4 is a control block diagram schematically illustrating a configuration of the main body M of the ultrasound imaging apparatus 1 according to an embodiment.

Referring to FIG. 4, the ultrasound imaging apparatus 1 according to the embodiment may include the ultrasound probe p, the sensor 300, the storage 560, the controller 500, the input 540, and the display 550.

The sensor 300 may acquire position information of the ultrasound probe p.

The position information of the ultrasound probe p may include the position of the ultrasound probe p with respect to a specific position, the movement speed of the ultrasound probe p, and the acceleration of the ultrasound probe p. In detail, the sensor 300 may include an acceleration sensor that measures the movement of the ultrasound probe p.

The acceleration sensor may be provided at a position adjacent to the transducer of the ultrasound probe p to predict the movement of the transducer. The position of the acceleration sensor is not limited thereto as long as it can measure the movement acceleration of the ultrasound probe p to predict the movement of the transducer.

In addition, the sensor 300 may be provided as a magnetic sensor.

The magnetic sensor may refer to a sensor that measures the magnitude and direction of a magnetic field or a line of magnetic force. The magnetic sensor measures the magnetic field on the basis of a change of properties of a substance due to the influence of the magnetic field. On the basis of the above-described operation, the magnetic sensor may measure the magnitude and direction of the line of magnetic force. The magnetic sensor may measure a movement of the probe on the basis of a change in the magnitude and direction of the line of magnetic force that is generated by the probe or modified due to a movement of the probe. The magnetic sensor may also include a magnetic field generator for generating a magnetic field.

The input 540 may receive a region of interest (ROI) of a user from the user.

The ROI may refer to an area that a user may designate to intensively diagnose an object.

The display 550 may output an ultrasound image determined throughout the specification.

The storage 560 may store a model of an object that is previously derived.

The model of the object may be formed on the basis of an image of the object.

The image of the object may be a cross sectional image of the object obtained by computed tomography (CT) imaging.

In more detail, the model of the object may be implemented as a 3D model formed on the basis of an image of the object formed of a tomography image of the object previously acquired.

The storage 560 may include a nonvolatile memory device, such as a cache, a read only memory (ROM), a programmable ROM (PROM), an erasable programmable ROM (EPROM), an electrically erasable programmable ROM (EEPROM), and a flash memory, a volatile memory device, such as a random access memory (RAM), or other storage media, such as a hard disk drive (HDD), a CD-ROM, and the like., but the implementation of the storage 560 is not limited thereto. The storage 560 may be a memory implemented as a chip separated from the processor, which has been described below in connection with the controller 500, or may be implemented as a single chip integrated with the processor.

The controller 500 determines an area of the model corresponding to the position of the ultrasound probe p when acquiring an ultrasound image of the object as a scan area.

The controller 500 may determine the position of the ultrasound probe p on the basis of the sensor 300 described above, and may determine an area of the model as a scan area on the basis of the positional relationship between the object and the ultrasound probe p.

The scan area may refer to an area of the model corresponding to an ultrasound image of the object acquired by the user using the ultrasound probe p.

In addition, the controller 500 may determine a diagnosis area used for diagnosing the object among the scan areas on the basis of at least one of a quality parameter of the ultrasound image or position information of the ultrasound probe p.

The diagnosis area may refer to an area used by the user to diagnose a disease of the object.

The quality parameter may include a transmission/reception parameter and a post-processing parameter of an ultrasound signal.

The post-processing parameter may include an image definition, a spatial compounding image (SCI) index, an imaging filter index, a spatial filter index, and the like.

In addition, when a real-time scan image and an ultrasound still image are implemented as a color image, the image quality parameter may include an ensemble value.

In detail, in order for an ultrasound image to be used for diagnosis of a disease of an object, the ultrasound image needs to be an ultrasound image obtained when the movement speed of the ultrasound probe p is less than a predetermined value such that the object is observed in detail, and when the quality of the acquired ultrasound image is high enough to facilitate disease determination.

The controller 500 determines the speed of the ultrasound probe p on the basis of the position information of the ultrasound probe p obtained from the sensor 300, and determines the quality of the ultrasound image on the basis of the quality parameter to determine whether the corresponding ultrasound image corresponds to a diagnosis area.

The controller 500 outputs the scan area and the diagnosis area to the display 550 such that the scan area and the diagnosis area correspond to the model.

The controller 500 determines an area of the model corresponding to the position of the ultrasound probe p when the movement speed of the ultrasound probe p is less than a predetermined value as the diagnosis area.

As described above, the movement speed of the ultrasound probe p may be included in the position information of the ultrasound probe p obtained by the sensor 300.

The controller 500, in response to the movement speed of the ultrasound probe p being less than the predetermined speed, may determine that the user acquires an ultrasound image for diagnosing the object, and may determine the diagnosis area on the basis of the positional relation between the ultrasound probe p and the mode when acquiring the ultrasound image.

The controller 500 may determine an area of the model corresponding to the position of the ultrasound probe p when the proportion of shade included in the ultrasound image acquired by the ultrasound probe p is less than a predetermined value as the diagnosis area.

The controller 500, in response to a lot of shadows existing in the ultrasound image, may determine that the quality of the acquired ultrasound image is poor and determine that the ultrasound image is difficult to be used for diagnosis.

The controller 500 may output the scan area and the diagnosis area to the display 550 such that each of the scan area and the diagnosis area corresponds to the model in a different form. Details thereof will be described below.

The controller 500 may learn the type of the object and the diagnosis area corresponding to the model and determine a reference diagnosis area corresponding to the object.

The reference diagnosis area is a portion of an area of the model of the object used for diagnosing a disease in the object and may refer to a reference area on which the user may acquire an ultrasound image and diagnose an object disease.

For example, the user may diagnose the object on the basis of an ultrasound image acquired by placing the ultrasound probe p to correspond to the obtained reference diagnosis area.

Meanwhile, the controller 500 may use a neural network to derive the reference diagnosis area. Neural networks are electronic networks constructed by mimicking human brain neurons and the associated structures, which may be used for type identification or computation of nonlinear mapping. Details thereof will be described below.

The controller 500 may output the model of the object to correspond to the reference diagnosis area as described above and guide the position of the ultrasound probe p to correspond to the reference diagnosis area on the basis of the positional relationship between the object and the ultrasound probe p.

That is, the controller 500 outputs the reference diagnosis area corresponding to the model of the object, thereby guiding the user to place the ultrasound probe p in the corresponding area and thus rapidly diagnosing the disease of the object.

The storage 560 may store at least one image of the object used to derive the model of the object. The image of the object may include a cross-sectional image of the object obtained by CT imaging as described above.

The controller 500 may output at least one of the ultrasound image, the model, and the object image corresponding to the ROI of the user.

The controller 500 may determine an area of the ultrasound image corresponding to the ROI set in the object image as an object area. The controller 500 may map the ROI input by the user in the object image to the ultrasound image, and determine an area of the ultrasound image corresponding to the ROI as the object area.

At least one component may be added or omitted to correspond to the performances of the components of the ultrasound imaging apparatus shown in FIG. 4. In addition, the mutual positions of the components may be changed to correspond to the performance or structure of the system.

The components shown in FIG. 4 may refer to a software component and/or a hardware component, such as a Field Programmable Gate Array (FPGA) and an Application Specific Integrated Circuit (ASIC).

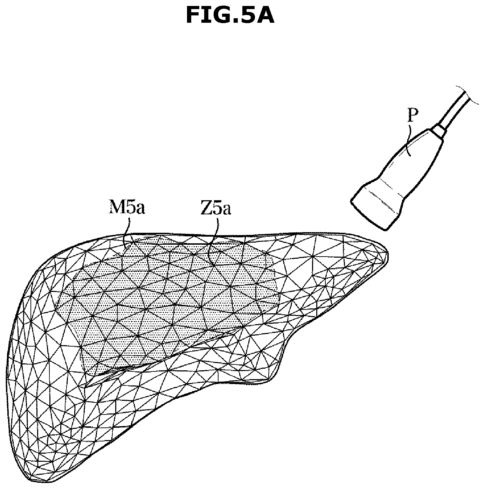

FIG. 5A is a diagram for describing a scan area according to an embodiment.

Referring to FIG. 5A, the storage may form an object model M5a in advance and determine a scan area on the basis of position information of the probe. As described above, the scan area may be formed by matching the position information of the probe with the model stored in advance.

That is, a scan area Z5a illustrated in FIG. 5A may refer to a portion of the object corresponding to an ultrasound image acquired by the user using the probe. The controller may determine a portion of the object acquired by the user through the probe, as the scan area Z5a.

In addition, the controller may output the above-described scan area together with the ultrasound image, to output an image related to the scan area and a change amount of the scan area in real time.

Meanwhile, the scan area may be distinguished from a diagnosis area described below.

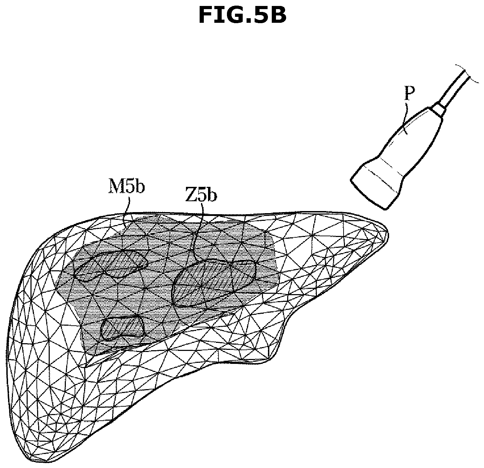

FIG. 5B is a diagram for describing a diagnosis area according to an embodiment.

Referring to FIG. 5B, the storage may form an object model M5b in advance and determine a scan area on the basis of position information of the probe. As described above, the scan area may be formed by matching the position information of the probe with the model stored in advance.

In addition, the controller may determine a diagnosis area Z5b used by the user to diagnose a disease of the object.

Meanwhile, the diagnosis area Z5b may be determined by position information of the probe and quality parameters of the ultrasound image. That is, the controller may determine whether the user has used the ultrasound image to diagnose the disease of the object on the basis of the position information of the probe.

For example, the user may determine an area corresponding to when the speed of the probe included in the position information of the probe among the positions of the probe corresponding to the scan areas is less than a predetermined value, as a diagnosis area used for diagnosing the object.

On the other hand, when the probe has a position corresponding to the above described scan area but having a movement speed exceeding the predetermined speed, an area corresponding to the position is not determined to a diagnosis area because it is determined that the object is simply scanned.

Also, the quality parameter may be used to determine the diagnosis area among the scan areas.

The quality parameter may refer to information obtained by quantifying the quality of the ultrasound image acquired by the probe.

The controller determines an area corresponding to when the speed of the probe included in the position information of the probe among the positions of the probe corresponding to the scan areas is less than the predetermined value, as the diagnosis area having been used for diagnosing the object.

However, when the identification of the object is not allowable because the image quality of the corresponding area is not good, the controller may not determine the corresponding area as the diagnosis area even when the movement speed of the probe corresponding to the area is less than the predetermined speed.

The controller may determine the diagnosis area on the basis of a proportion of shadow included in the ultrasound image acquired by the probe.

In more detail, when a large amount of shadow is included in the corresponding area, and thus the identification of the object is impossible, the controller may not determine the corresponding area as the diagnosis area even when the movement speed of the probe corresponding to the area is less than the predetermined speed.

The controller may output the above-described diagnosis area together with the ultrasound image, to output an image related to the diagnosis area and a change amount of the diagnosis area in real time.

Meanwhile, each of the scan area Z5a and the diagnosis area Z5b shown in FIGS. 5A and 5B may be displayed on the display in a different form.

Meanwhile, the operation of displaying the scan area and the diagnosis area described with reference to FIGS. 5A and 5B corresponds to an embodiment of the present disclosure, and it is not limited thereto.

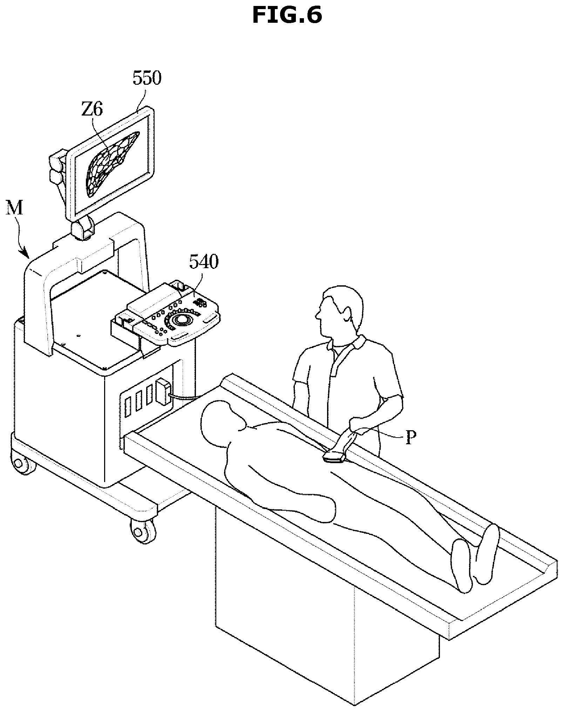

FIG. 6 is a diagram for describing an operation of guiding a user on the basis of a reference diagnosis area according to an embodiment.

Referring to FIG. 6, the controller may determine a reference diagnosis area Z6 on the basis of the above-described operation. The reference diagnosis area Z6 may refer to an area of a model determined by accumulating diagnosis areas used for diagnosing a disease of an object.

Meanwhile, the controller may output the reference diagnosis area on the display, and the user may diagnose the object by placing the probe to correspond to the reference diagnosis area.

In the conventional technology, an object is scanned using a probe and an area required for diagnosis is detected from the scanned areas to be used to diagnose the object. However, according to the present disclosure, the controller outputs the reference diagnosis area in advance and guides the position of the probe such that the user places the probe to correspond to the reference diagnosis area. In this case, the controller may use the position of the probe acquired by the sensor.

In the present disclosure, the controller performs learning using data manually obtained by an expert from diagnosis areas accumulated in acquired ultrasound images as correct answer data.

The input of the controller may be provided as an ultrasound image and position information of the probe. In addition, the output value of the controller may be provided as a reference diagnosis area.

The controller when using the neural network may use a binary image, which is a scan of a liver area in an ultrasound image as an input, so that the performance of the neural network is improved.

The above operation may prevent an error that an area other than the object area is determined to be a diagnosis area, and may improve the accuracy of distinguishing the area used for diagnosis within the object.

The controller may derive the positional relationship between the object and the probe, and guide the position of the probe to correspond to the reference diagnosis area. Through such a guide, the user may diagnose a disease of the object more rapidly and accurately compared to the conventional technology.

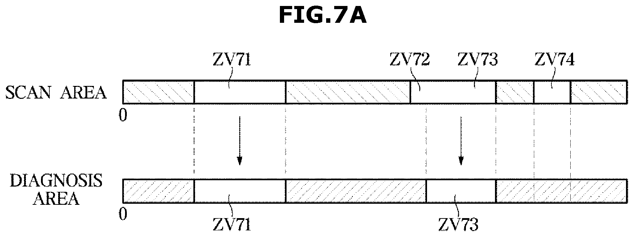

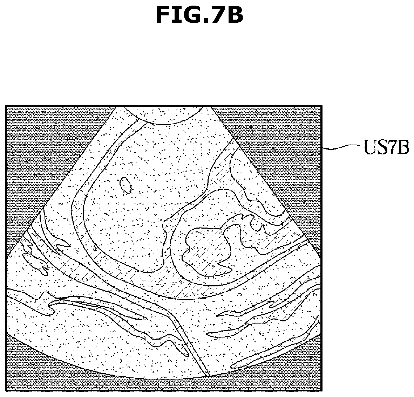

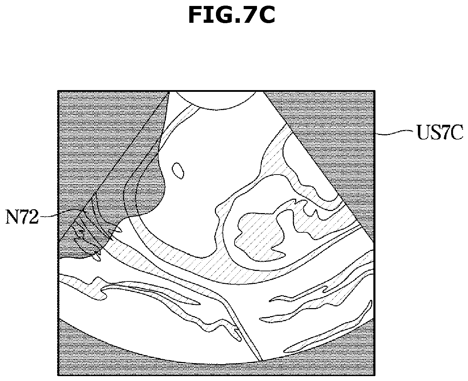

FIG. 7A is a diagram for describing an operation of determining a scan area and a diagnosis area according to an embodiment, FIG. 7B is a diagram for describing a relationship between an ultrasound image and an image quality according to an embodiment, and FIG. 7C is a diagram for describing a relationship between an ultrasound image and shadow according to an embodiment.

In FIG. 7A, a scan area and a diagnosis area of a model of an object are schematically illustrated.

The respective bars shown in FIG. 7A may refer to the entire model of the object.

Meanwhile, ZV71, ZV72, ZV73, and ZV74 may correspond to portions of the object scanned by a user using a probe.

In detail, the corresponding areas ZV71, ZV72, ZV73, and ZV74 may refer to portions in which the probe has scanned the object at a speed less than a predetermined speed. The controller may determine the portions as portion in which the probe has been moved slowly.

Referring to FIGS. 7A and 7B, ZV72 is a portion corresponding to a fatty liver area and in which an ultrasound image US7B is acquired at a low quality. That is, the controller may determine that ZV72 is a low quality ultrasound image on the basis of the quality parameter of the ultrasound image corresponding to ZV72, and exclude the corresponding area from the diagnosis area.

Referring to FIGS. 7A and 7C, an area ZV74 may refer to a case in which a large amount of shades N7C are included in an ultrasound image US7C. In this case, the controller may determine that the area ZV74 may be difficult to identify a disease of the object due to having a large amount of shadows although the area ZV74 has been scanned at a speed lower than a predetermined speed, and thus may exclude the area ZV74 from the diagnosis area.

Accordingly, the controller may determine the areas ZV71 and ZV73 in the areas ZV71, ZV72, ZV73, and ZV74 except for the areas ZV72 and ZV74, as diagnosis areas.

Meanwhile, the operations described with reference to FIGS. 7A to 7C are merely examples for describing the difference between the scan area and the diagnosis area of the present disclosure, and are not intended to limit the operation of the present disclosure.

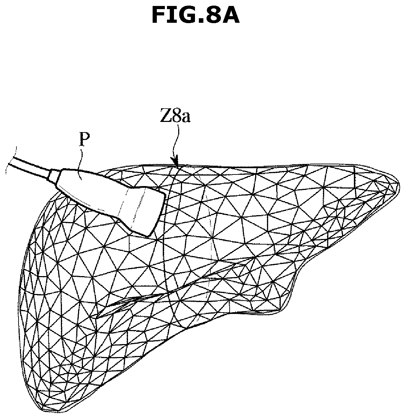

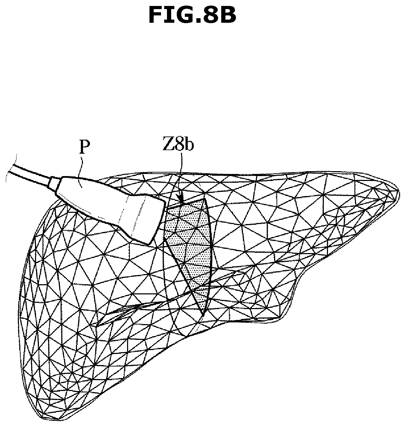

FIG. 8A is a diagram for describing an operation of outputting a scan area according to an embodiment, and FIG. 8B is a diagram for describing an operation of outputting a diagnosis area according to an embodiment.

Referring to FIGS. 8A and 8B, the operations of outputting a scan area Z8a and a diagnosis area Z8b of a model in real time are described.

The controller may match the position information of the probe acquired by the sensor with the model, to output the portions Z8a and Z8b of the object corresponding to the ultrasound image currently acquired by the user in real time.

As described above, since the scan area Z8a and the diagnosis area Z8b are distinguished, each of the scan area and the diagnosis area may be output to correspond to the model in a different form on the display.

In detail, a portion for which the user has simply scanned and obtained an ultrasound image of the object may be output to the display as the scan area Z8a, and a portion determined to have been used for diagnosing a disease of the object on the basis of the movement speed of the probe and the quality of the ultrasound image may be determined as the diagnosis area Z8b and output on the display.

Meanwhile, in FIG. 8A and 8B, the form of displaying the scan area and the diagnosis area is illustrated only as one embodiment of the present disclosure, and there is no limitation in the form of the output of the controller.

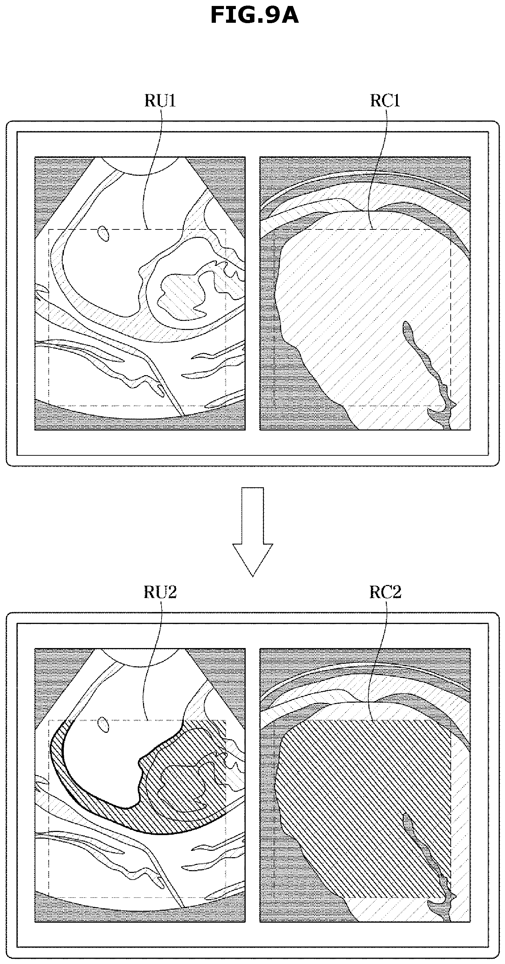

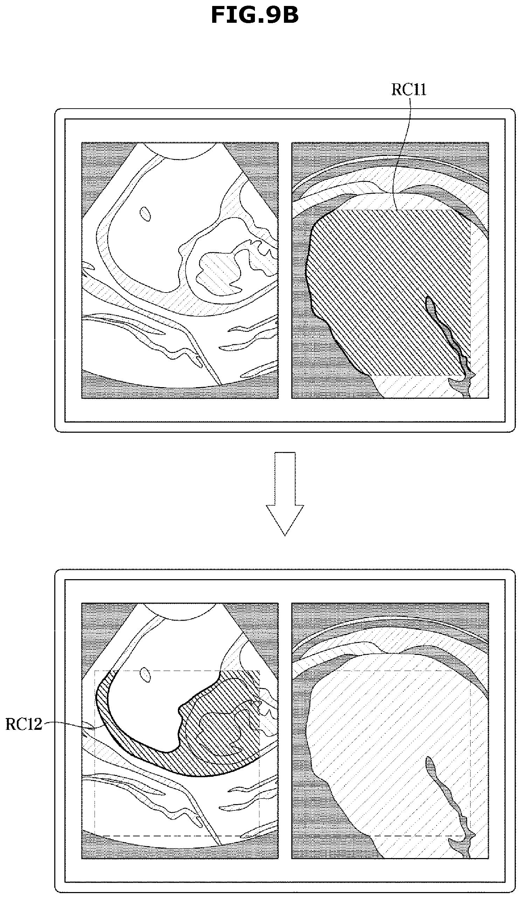

FIGS. 9A and 9B are diagrams for describing an operation associated with an ROI according to an embodiment.

FIG. 9A illustrates a case in which a user inputs an ROI. According to the embodiment, the user may input an ROI RU1 in an ultrasound image and may input an ROI RC1 in an object image. The controller may output portions RU2 and RC2 of the actual object corresponding to the ROIs on the basis of the ROIs input by the user.

FIG. 9B illustrates an operation in which the user inputs an ROI RC11 in the object image.

The controller may determine an area of the ultrasound image corresponding to the ROI RC11 set in the object image as an object area RC12. That is, the user may determine the object area RC12 by matching the area set in the object image with the area set in the actual ultrasound area, and output the object area RC12 on the display together the object image.

Meanwhile, although FIGS. 9A and 9B have been described on the basis of the object image and the ultrasound image, the controller may match the ROI with the model, and there is no limitation in the ROI that is set by the user and output in a correspondence form.

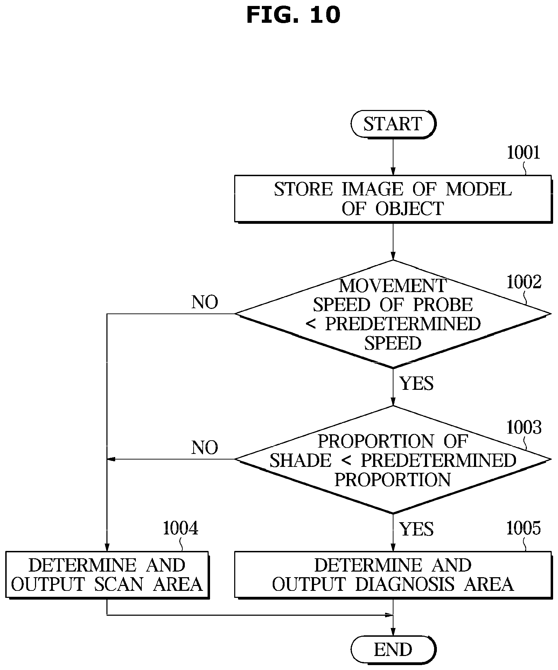

FIG. 10 is a flowchart according to an embodiment.

Referring to FIG. 10, the ultrasound imaging apparatus may store a model of an object in advance (1001).

Meanwhile, the ultrasound imaging apparatus may determine a scan area and a diagnosis area on the basis of the quality parameter of the ultrasound image and the position information of the probe.

When the movement speed of the probe is less than a predetermined speed (1002), and the proportion of shade included in the ultrasound image is less than a predetermined proportion (1003), the ultrasound imaging apparatus may determine the corresponding area as a diagnosis area and output the diagnosis area on the display (1005).

On the other hand, when the movement speed of the probe exceeds the predetermined speed (1002), or when the movement speed of the probe is less than the predetermined speed but the proportion of shade included in the ultrasound image exceeds the predetermined proportion (1003), the ultrasound imaging apparatus may determine the corresponding area as a scan area and output the scan area on the display (1004).

Meanwhile, the disclosed embodiments may be embodied in the form of a recording medium storing instructions executable by a computer. The instructions may be stored in the form of program code and, when executed by a processor, may generate a program module to perform the operations of the disclosed embodiments. The recording medium may be embodied as a computer-readable recording medium.

The computer-readable recording medium includes all kinds of recording media in which instructions which may be decoded by a computer are stored, for example, a Read Only Memory (ROM), a Random Access Memory (RAM), a magnetic tape, a magnetic disk, a flash memory, an optical data storage device, and the like.

As is apparent from the above, the ultrasound imaging apparatus and the method of controlling the same can enhance the diagnosis efficiency of an object by learning an area used for a diagnosis of an object and providing information about the area at a later time.

Although embodiments of the present disclosure have been described for illustrative purposes, those skilled in the art will appreciate that various modifications, additions and substitutions are possible, without departing from the scope of the disclosure. Therefore, embodiments of the present disclosure have not been described for limiting purposes.

## Claims

1. An ultrasound imaging apparatus (1) comprising:
an output:
a probe (P);
a sensor (300) configured to acquire position information of the probe;
a storage configured to store a model of an object that is derived in advance; and
a controller (500) configured to:
determine an area of the model corresponding to a position of the probe when acquiring an ultrasound image of the object as a scan area;
determine a diagnosis area (Z8b) used for diagnosis of the object among the scan areas (ZV71, ZV72, ZV73, ZV74, Z8a) on the basis of at least one of a quality parameter of the ultrasound image or the position information of the probe; and
output the scan area and the diagnosis area to correspond to the model on the output. **characterized in that**
the controller determines the area of the model corresponding to the position of the probe when a movement speed of the probe is less than a predetermined value as the diagnosis area.

2. The ultrasound imaging apparatus (1) of claim 1, wherein the controller determines the area of the model corresponding to the position of the probe when a proportion of shade included in the ultrasound image acquired by the probe is less than a predetermined value as the diagnosis area.

3. The ultrasound imaging apparatus (1) of claim 1, wherein the controller outputs each of the scan area and the diagnosis area to correspond to the object in a different form.

4. The ultrasound imaging apparatus (1) of claim 1, wherein the controller learns a type of the object and the diagnosis area corresponding to the model, and determines a reference diagnosis area corresponding to the object.

5. The ultrasound imaging apparatus (1) of claim 4, wherein the controller outputs the reference diagnosis area to correspond to the model of the object, and
guide the position of the ultrasound probe (P) to match with the reference diagnosis area on the basis of a positional relationship between the object and the probe.

6. The ultrasound imaging apparatus (1) of claim 1, further comprising an input (540) configured to receive a region of interest, ROI, of a user,
wherein the storage stores at least one image of the object used for deriving the model, and
the controller outputs at least one of the ultrasound image, the model or the image of the object corresponding to the ROI of the user.

7. The ultrasound imaging apparatus (1) of claim 6, wherein the controller determines an area of the ultrasound image corresponding to the ROI set in the image of the object as an object area.

8. A computer-implemented method for controlling an ultrasound imaging apparatus, the method comprising:
acquiring position information of a probe (P);
storing a model of an object that is derived in advance;
determining an area of the model corresponding to a position of the probe when acquiring an ultrasound image of the object as a scan area;
determining a diagnosis area (Z8b) used for diagnosis of the object among the scan areas (ZV71, ZV72, ZV73, ZV74, Z8a) on the basis of at least one of a quality parameter of the ultrasound image or the position information of the probe; and
outputting the scan area and the diagnosis area to correspond to the model.
wherein the determining of the diagnosis area comprises
**characterized by**
determining the area of the model corresponding to the position of the probe when a movement speed of the probe is less than a predetermined value as the diagnosis area.

9. The method of claim 8, wherein the determining of the diagnosis area comprises determining the area of the model corresponding to the position of the probe when a proportion of shade included in the ultrasound image acquired by the probe is less than a predetermined value as the diagnosis area.

10. The method of claim 8, wherein the outputting of the scan area and the diagnosis area to correspond to the model comprises
outputting each of the scan area and the diagnosis area to correspond to the object in a different form.

11. The method of claim 8, further comprising learning a type of the object and the diagnosis area corresponding to the model, and determining a reference diagnosis area corresponding to the object.

12. The method of claim 11, further comprising:
outputting the reference diagnosis area to correspond to the model of the object; and
guiding the position of the ultrasound probe p to match with the reference diagnosis area on the basis of a positional relationship between the object and the probe.

13. The method of claim 8, further comprising:
receiving a region of interest, ROI, of a user;
storing at least one image of the object used for deriving the model; and
outputting at least one of the ultrasound image, the model or the image of the object corresponding to the ROI of the user.

## Patentansprüche

1. Ultraschallbildgebungsvorrichtung (1), die Folgendes umfasst:
eine Ausgabevorrichtung;
eine Sonde (P);
einen Sensor (300), der so konfiguriert ist, dass er Informationen über die Position der Sonde erhält;
einen Speicher, der so konfiguriert ist, dass er ein Modell eines Objekts speichert, das im Voraus abgeleitet wird; und
eine Steuerung (500), die zu Folgendem konfiguriert ist:
Bestimmen eines Bereichs des Modells, der einer Position der Sonde entspricht, wenn ein Ultraschallbild des Objekts erhalten wird, als Scanbereich;
unter den Scanbereichen (ZV71, ZV72, ZV73, ZV74, Z8a), Bestimmen eines Diagnosebereichs (Z8b), der für die Diagnose des Objekts verwendet wird, basierend auf einem Qualitätsparameter des Ultraschallbildes und/oder den Informationen über die Position der Sonde; und
Ausgeben des Scanbereichs und des Diagnosebereichs, so dass sie dem Modell entsprechen, in der Ausgabevorrichtung;
**dadurch gekennzeichnet, dass** die Steuerung den Bereich des Modells, der der Position der Sonde entspricht, wenn eine Bewegungsgeschwindigkeit der Sonde kleiner als ein vorbestimmter Wert ist, als Diagnosebereich bestimmt.

2. Ultraschallbildgebungsvorrichtung (1) nach Anspruch 1, wobei die Steuerung den Bereich des Modells, der der Position der Sonde entspricht, wenn ein in dem von der Sonde erhaltenen Ultraschallbild eingeschlossener Schattenanteil kleiner als ein vorbestimmter Wert ist, als Diagnosebereich bestimmt.

3. Ultraschallbildgebungsvorrichtung (1) nach Anspruch 1, wobei die Steuerung sowohl den Scanbereich als auch den Diagnosebereich so ausgibt, dass sie dem Objekt in unterschiedlicher Form entsprechen.

4. Ultraschallbildgebungsvorrichtung (1) nach Anspruch 1, wobei die Steuerung eine Art des Objekts und den dem Modell entsprechenden Diagnosebereich lernt und einen dem Objekt entsprechenden Referenzdiagnosebereich bestimmt.

5. Ultraschallbildgebungsvorrichtung (1) nach Anspruch 4, wobei die Steuerung den Referenzdiagnosebereich so ausgibt, dass er dem Modell des Objekts entspricht, und die Position der Ultraschallsonde (P) basierend auf einer Positionsbeziehung zwischen dem Objekt und der Sonde so ausrichtet, dass sie mit dem Referenzdiagnosebereich übereinstimmt.

6. Ultraschallbildgebungsvorrichtung (1) nach Anspruch 1, ferner umfassend eine Eingabevorrichtung (540), die so konfiguriert ist, dass sie eine Region von Interesse (ROI, Region of Interest) eines Benutzers empfängt,
wobei der Speicher zumindest ein Bild des Objekts speichert, das zur Ableitung des Modells verwendet wird, und
die Steuerung zumindest eines von dem Ultraschallbild, dem Modell oder dem Bild des Objekts, das der ROI des Benutzers entspricht, ausgibt.

7. Ultraschallbildgebungsvorrichtung (1) nach Anspruch 6, wobei die Steuerung einen Bereich des Ultraschallbildes, der der in dem Bild des Objekts festgelegten ROI entspricht, als Objektbereich bestimmt.

8. Computerimplementiertes Verfahren zum Steuern einer Ultraschallbildgebungsvorrichtung, wobei das Verfahren Folgendes umfasst:
Erhalten von Informationen über die Position einer Sonde (P);
Speichern eines Modells eines Objekts, das im Voraus abgeleitet wird;
Bestimmen eines Bereichs des Modells, der einer Position der Sonde entspricht, wenn ein Ultraschallbild des Objekts erhalten wird, als Scanbereich;
unter den Scanbereichen (ZV71, ZV72, ZV73, ZV74, Z8a), Bestimmen eines Diagnosebereichs (Z8b), der für die Diagnose des Objekts verwendet wird, basierend auf einem Qualitätsparameter des Ultraschallbildes und/oder den Informationen über die Position der Sonde; und
Ausgeben des Scanbereichs und des Diagnosebereichs, so dass sie dem Modell entsprechen;
**dadurch gekennzeichnet, dass** das Bestimmen des Diagnosebereichs Folgendes umfasst: Bestimmen des Bereichs des Modells, der der Position der Sonde entspricht, wenn eine Bewegungsgeschwindigkeit der Sonde kleiner als ein vorbestimmter Wert ist, als Diagnosebereich.

9. Verfahren nach Anspruch 8, wobei das Bestimmen des Diagnosebereichs Folgendes umfasst: Bestimmen des Bereichs des Modells, der der Position der Sonde entspricht, wenn ein in dem von der Sonde erhaltenen Ultraschallbild eingeschlossener Schattenanteil kleiner als ein vorbestimmter Wert ist, als Diagnosebereich.

10. Verfahren nach Anspruch 8, wobei das Ausgeben des Scanbereichs und des Diagnosebereichs, so dass sie dem Modell entsprechen, das Ausgeben sowohl des Scanbereichs als auch des Diagnosebereichs so umfasst, dass sie dem Objekt in einer unterschiedlichen Form entsprechen.

11. Verfahren nach Anspruch 8, ferner umfassend das Lernen einer Art des Objekts und des dem Modell entsprechenden Diagnosebereichs, sowie das Bestimmen eines Referenzdiagnosebereichs, der dem Objekt entspricht.

12. Verfahren nach Anspruch 11, das ferner Folgendes umfasst:
Ausgeben des Referenzdiagnosebereichs, so dass er dem Modell des Objekts entspricht; und
Ausrichten der Position der Ultraschallsonde (P), so dass sie mit dem Referenzdiagnosebereich übereinstimmt, basierend auf einer Positionsbeziehung zwischen dem Objekt und der Sonde.

13. Verfahren nach Anspruch 8, das ferner Folgendes umfasst:
Empfangen einer Region von Interesse (ROI, Region of Interest) eines Benutzers;
Speichern zumindest eines Bildes des Objekts, das zur Ableitung des Modells verwendet wird; und
Ausgeben zumindest eines von dem Ultraschallbild, dem Modell oder dem Bild des Objekts, das der ROI des Benutzers entspricht.

## Revendications

1. Appareil échographique (1) comprenant :
un dispositif émetteur,
une sonde (P),
un capteur (300) conçu pour acquérir des informations de position concernant la sonde,
un dispositif de stockage conçu pour stocker un modèle d'un objet dérivé à l'avance, et
un organe de commande (500) conçu pour :
déterminer comme zone à échographier une zone du modèle correspondant à la position de la sonde lors de l'acquisition d'une image échographique de l'objet,
déterminer une zone de diagnostic (Z8b) servant au diagnostic de l'objet, parmi les zones à échographier (ZV71, ZV72, ZV73, ZV74, Z8a), compte tenu d'un paramètre de qualité de l'image échographique et/ou des informations de position de la sonde, et
émettre la zone à échographier et la zone de diagnostic de façon qu'elles correspondent au modèle,
sur le dispositif émetteur ;
**caractérisé en ce que** l'organe de commande détermine comme zone de diagnostic la zone du modèle correspondant à la position de la sonde lorsque la vitesse de déplacement de la sonde est inférieure à une valeur prédéterminée.

2. Appareil échographique (1) selon la revendication 1, dans lequel l'organe de commande détermine comme zone de diagnostic la zone du modèle correspondant à la position de la sonde lorsqu'une proportion d'ombre présente dans l'image échographique acquise par la sonde est inférieure à une valeur prédéterminée.

3. Appareil échographique (1) selon la revendication 1, dans lequel l'organe de commande émet la zone à échographier et la zone de diagnostic de façon qu'elles correspondent chacune à l'objet sous une forme différente.

4. Appareil échographique (1) selon la revendication 1, dans lequel l'organe de commande apprend le type de l'objet et la zone de diagnostic correspondant au modèle, et détermine une zone de diagnostic de référence correspondant à l'objet.

5. Appareil échographique (1) selon la revendication 4, dans lequel l'organe de commande émet la zone de diagnostic de référence de façon qu'elle corresponde au modèle de l'objet, et guide la position de la sonde à ultrasons (P) de façon qu'elle corresponde à la zone de diagnostic de référence compte tenu d'une relation de position entre l'objet et la sonde.

6. Appareil échographique (1) selon la revendication 1, comprenant en outre un dispositif d'entrée (540) conçu pour recevoir une région d'intérêt (ROI, region of interest) d'un utilisateur,
ledit dispositif de stockage stockant au moins une image de l'objet servant à dériver le modèle, et
l'organe de commande émettant l'image échographique, le modèle et/ou l'image de l'objet correspondant à la ROI de l'utilisateur.

7. Appareil échographique (1) selon la revendication 6, dans lequel l'organe de commande détermine, en tant que zone d'objet, une zone de l'image échographique correspondant à la ROI définie dans l'image de l'objet.

8. Procédé mis en oeuvre par ordinateur pour commander un appareil échographique, le procédé comprenant les opérations consistant à :
acquérir des informations de position concernant une sonde (P),
stocker un modèle d'un objet dérivé à l'avance,
déterminer, en tant que zone à échographier, une zone du modèle correspondant à la position de la sonde lors de l'acquisition d'une image échographique de l'objet,
déterminer une zone de diagnostic (Z8b) servant au diagnostic de l'objet, parmi les zones à échographier (ZV71, ZV72, ZV73, ZV74, Z8a), compte tenu d'un paramètre de qualité de l'image échographique et/ou des informations de position de la sonde, et
émettre la zone à échographier et la zone de diagnostic de façon qu'elles correspondent au modèle ;
**caractérisé en ce que** la détermination de la zone de diagnostic comprend la détermination, comme zone de diagnostic, de la zone du modèle correspondant à la position de la sonde lorsque la vitesse de déplacement de la sonde est inférieure à une valeur prédéterminée.

9. Procédé selon la revendication 8, dans lequel la détermination de la zone de diagnostic comprend la détermination, comme zone de diagnostic, de la zone du modèle correspondant à la position de la sonde lorsqu'une proportion d'ombre présente dans l'image échographique acquise par la sonde est inférieure à une valeur prédéterminée.

10. Procédé selon la revendication 8, dans lequel l'émission de la zone à échographier et de la zone de diagnostic de façon qu'elles correspondent au modèle comprend l'émission de la zone à échographier et de la zone de diagnostic de façon qu'elles correspondent chacune à l'objet sous une forme différente.

11. Procédé selon la revendication 8, comprenant en outre l'apprentissage du type de l'objet et de la zone de diagnostic correspondant au modèle, et la détermination d'une zone de diagnostic de référence correspondant à l'objet.

12. Procédé selon la revendication 11, comprenant en outre les opérations consistant à :
générer la zone de diagnostic de référence de façon qu'elle corresponde au modèle de l'objet, et
guider la position de la sonde à ultrasons (P) de façon qu'elle corresponde à la zone de diagnostic de référence compte tenu d'une relation de position entre l'objet et la sonde.

13. Procédé selon la revendication 8, comprenant en outre les opérations consistant à :
recevoir une région d'intérêt (ROI, region of interest) d'un utilisateur,
stocker au moins une image de l'objet servant à dériver le modèle, et
émettre l'image échographique, le modèle et/ou l'image de l'objet correspondant à la ROI de l'utilisateur.
